# EUROPÄISCHE PATENTSCHRIFT

(11) **EP 1 188 434 B1**
(45) Veröffentlichungstag und Bekanntmachung des Hinweises auf die Patenterteilung: **17.05.2006**
(21) Anmeldenummer: 01121564.7
(22) Anmeldetag: 10.09.2001
(51) Int. Cl.: A61K 9/00

(54) **Verwendung einer wässrigen Lösung oder Suspension zum Heilen des Hornhautepithels**
Use of an aqueous solution or suspension for healing corneal epithelium
Utilisation d'une solution ou suspension aqueuse pour la guérison de l'épithélium cornéen

(30) Priorität: 14.09.2000 DE 10045343
(43) Veröffentlichungstag der Anmeldung: 20.03.2002
(73) Patentinhaber: Neuhann, Tobias Dr. med., 80803 München (DE)
(72) Erfinder: Neuhann, Tobias Dr. med., 80803 München (DE)
(74) Vertreter: Nöth, Heinz

(56) Entgegenhaltungen:
- WO-A-92/15614
- WO-A-95/20969
- US-A- 5 104 787

## Beschreibung

Die Erfindung betrifft die Verwendung einer wässrigen Lösung oder Suspension, welche begleitend bei refraktiver Hornhautchirurgie oder zur Hornhauttransplantation einsetzbar ist.

Aus WO 95/20969 ist eine mit der Bezeichnung Optisol® im Handel befindliche Konservierungslösung bekannt, mit der menschliche Hornhäute über mehrere Wochen so aufbewahrt werden können, dass sie für die Transplantation geeignet bleiben. Dadurch ist es möglich, Hornhautbanken zu installieren und somit den weltweit stetigen Bedarf an Transplantationsmaterial organisatorisch zu koordinieren. Mit derartigen Konservierungslösungen wird einmal die Autolyse (Zerfall nach dem Tod) erheblich hinausgezögert und zum anderen das Absterben des empfindlichen Endothels deutlich verzögert.

Derartige Konservierungslösungen enthalten in der Regel Zucker, insbesondere Dextran oder Glucose, Vitamine, messentielle und nicht-essentielle Aminosäuren, Elektrolyte, Eisenverbindungen, ein Puffersystem, ein Chondroitinsulfat, L-Hydroxyprolin, Adenosin, Adenin, Lactoferrin und Lactoperoxydase sowie ein oder mehrere Antibiotika, z.B. Gentamycin und Streptomycin. Als Puffer wird häufig HEPES, d.h. N-2-Hydroxyethylpiperazin-N-2-ethansulfonsäure eingesetzt.

Vor allem in jüngerer Zeit wird zur Behebung von Sehschwächen vermehrt refraktive Hornhautchirurgie wie insbesondere Lasik angewendet. Zur begleitenden Behandlung und insbesondere zur Nachbehandlung bei refraktiver Hornhautchirurgie gibt es derzeit kein zufriedenstellendes Augenarzneimittel.

Aufgabe der Erfindung ist es, die Herstellung eines Mittels zu schaffen, mit dem die Nachbehandlung bei refraktiver Hornhautchirurgie oder Hornhauttransplantation verbessert wird.

Diese Aufgabe wird gemäß Patentanspruch 1 gelöst.

Die Lösung der Aufgabe beruht auf dem überraschenden Befund, daß bestimmte wäßrige Lösungen bzw. Suspensionen wie sie bereits als Konservierungslösungen für entnommene menschliche Hornhäute bekannt sind, eine ausgezeichnete Wirksamkeit zeigen.

Die verwendete Lösung oder Suspension enthält zumindest ein Kohlenhydrat, insbesondere Glucose und/oder Dextran, ein oder mehrere essentielle oder nicht-essentielle Aminosäuren und ein Elektrolyt, das bevorzugt aus mehreren anorganischen Salzen besteht. Ferner kann darüber hinaus im allgemeinen auch noch zumindest ein Puffersystem enthalten sein, wobei HEPES (N-2-Hydroethylpiperazin-N-2-ethansulfonsäure) bevorzugt ist. Außerdem können bevorzugt enthalten sein ein oder mehrere Mittel ausgewählt aus Eisenverbindungen, L-Hydroxyprolin, Vorläuferstoffen für Adenosintriphosphat, insbesondere Adenosin, Inosin und Adenin, Proteinen wie Lactoferrin und Enzymen wie Lactoperoxydase. Auch weitere übliche Hilfsstoffe können gegebenenfalls vorhanden sein.

Bevorzugt wird als Elektrolyt ein anorganisches Salz oder ein Gemisch aus mehreren anorganischen Salzen, insbesondere aus drei oder mehr anorganischen Salzen eingesetzt. Geeignete anorganische Salze, die einzeln oder im Gemisch verwendet werden können sind beispielsweise Magnesiumsalze, Kaliumsalze, Selensalze, Natriumsalze etc., insbesondere beispielsweise Calciumchlorid, Kaliumchlorid, Magnesiumsulfat, Natriumchlorid, Natriumhydrogencarbonat, Natriumdihydrogenphosphat, Eisennitrat, etc. Geeignete Vitamine sind beispielsweise Vitamin B12, Biotin, Vitamin B6 etc. Bevorzugt enthalten die Mittel ein Gemisch aus mehreren, insbesondere drei oder mehr, stärker bevorzugt sieben oder mehr essentiellen und/oder nichtessentiellen Aminosäuren. Als geeignete Aminosäuren können alle in den verwendeten Konzentrationen pharmakologisch unbedenklichen Aminosäuren verwendet werden, beispielsweise L-Alanin, L-Arginin, L-Asparagin, L-Aspartat, L-Cystein, L-Glutaminsäure, L-Glutamin, L-Histidin, L-Isoleucin, L-Leucin, L-Lysin, L-Methonin, L-Phenylalanin, L-Prolin, L-Serin, L-Thyrosin, L-Tryptophan, L-Treonin, L-Valin, Glycin etc. Die Mischungsverhältnisse sind nicht kritisch.

Die verwendete Lösung oder Suspension ist frei von Steroiden mit Hormonwirkung. Allerdings können Verbindungen mit einem Steroidgerüst wie Cholesterin in geringen Mengen vorhanden sein. Daher treten die üblichen Nebenwirkungen steroidaler Augenarzneimittel, wie intraokulare Druckerhöhung nicht auf.

Bevorzugt wird eine wäßrige Lösung oder Suspension eingesetzt, wie sie bereits als Aufbewahrungslösung für humane Hornhauttransplantate bekannt und im Handel erhältlich ist.

Hierbei sind z.B. Aufbewahrungslösungen zu nennen, wie sie unter- der Bezeichnung POLYSOL® von der Polytech Ophthalmologie GmbH vertrieben und in öffentlich zugänglichen Verbrauchsinformationen beschrieben werden und die folgende Zusammensetzung aufweisen:
- Glucose
- Natriumpyruvat (1 mM)
- essentielle und nicht-essentielle Aminosäuren (1,74 g/L)
- Vitamine (B12, Biotin, B6 ...)
- Elektrolyte (Ca, Mg, K, Se ...)
- Gentamycin, Penicillin G, Streptomycin
- Chondroitinsulfat (2 %)
- Lactoferrin
- Lactoperoxydase
- HEPES und Bicarbonat-Puffer
- Phenolrot
pH 7,4
Osmolarität 320 mOsm/kg H₂O.

Als weiteres Beispiel können auch Formulierungen genannt werden, wie sie unter der Bezeichnung Likorol® von der Firma Laboratoires Opsia-Pharma Frankreich erhältlich sind. Die Likorol® -Lösung weist im wesentlichen die gleiche Zusammensetzung auf, wie das Polysol® -Produkt.

Von der Firma Opsia-Pharma sind ebenfalls die Produkte Inosol und Exosol erhältlich, die erfindungsgemäß ebenfalls einsetzbar sind. Die Zusammensetzung der Inosol-Lösung ist wie folgt:
- Zellulares Kulturmedium (IMDM)
- Fetales Kälberserum
- Glucose
- Natriumpyruvat (1mM)
- essentielle und nicht-essentielle Aminosäuren (1,74 g/l)
- Vitamine (B12, Biotin, Folsäure)
- Electrolyt (Ca++, Mg++, K+, Se+ ...)
- Penicillin, Streptomycin, Amphotericin B
- Antioxidationsmittel
- HEPES und Bicarbonatpuffer
- Phenol rot
pH = 7,4
Osmolarität = 320 mOsm/kg H₂O

Die Exosol-Lösung entspricht der Inosol-Lösung, nur daß sie zusätzlich noch Dextran enthält.

Besonders bevorzugt ist die unter der Bezeichnung Optisol kommerziell erhältliche Aufbewahrungslösung für Hornhäute, die beispielsweise im American Journal of Ophthalmology 114, 345-356, September 1992 beschrieben ist. Auf den Inhalt dieser Druckschrift wird insoweit Bezug genommen, insbesondere zur genauen Zusammensetzung der Optisol® Lösungen. Optisol® - Aufbewahrungslösungen für Hornhäute werden ebenfalls in der Druckschrift Arch Ophthalmol - Vol. 109, Juni 1991, 864-868 beschrieben, auf die ebenfalls Bezug genommen wird.

Für einen Fachmann ist es ohne weiteres möglich, einzelne Inhaltsstoffe der kommerziell erhältlichen Konservierungslösungen für entnommene menschliche Hornhäute durch gleichwertige Bestandteile auszutauschen und selbstverständlich sind auch derartig Lösungen erfindungsgemäß bevorzugt.

Die Dosierung hängt von der Indikation, dem Alter und Zustand des Patienten usw. ab und kann von dem behandelnden Arzt ohne Schwierigkeiten bestimmt werden. Wird beispielsweise die bevorzugte Optisollösung als Augenarzneimittel zur Nachbehandlung einer refraktiven Hornhautchirurgie mittels Lasik eingesetzt, werden 3 bis 4 mal täglich jeweils 1 Tropfen der Lösung an jedes zu behandelnde Auge verabreicht. Entsprechende Dosierungen sind auch für die anderen Indikationen bevorzugt.

Die wassrige Lösung oder Suspension wird als begleitende Medikation bei refraktiver Hornhautchirurgie, insbesondere bei Lasik, aber auch bei PRK und PTK verwendet. Die nach der Excimerlaserchirurgie häufig angegriffenen Hornhäute werden aufgehellt und die Heilung kleiner Epithelverletzungen wird begünstigt. Da das erfindungsgemäße Augenarzneimittel keine Steroide enthält, treten keine Nebenwirkungen auf, die üblicherweise bei steroidalen Arzneimitteln auftreten wie z.B. eine intraokulare Druckerhöhung.

Ein weiterer Anwendungsbereich der wassrigen Lösung oder Suspension ist bei Hornhauttransplantationen, wobei das Mittel postoperativ als Augentropfen geeignet ist.

Die folgenden Beispiele erläutern die Erfindung.

### Beispiel 1

Bei einer 81-jährigen Patientin war wegen Eintrübung eines ersten Hornhauttransplantates nach sechs Jahren eine erneute Hornhauttransplantation notwendig geworden. Die Ursache für die Eintrübung war nicht klar, da die objektiven Daten des ersten Transplantates gut waren. Nach dem ersten Implantat war ein stationärer Aufenthalt nach der Operation über 17 Tage notwendig, d.h. die postoperative Einheilung erfolgte langsam.

Bei der erneuten Hornhauttransplantation wurde eine postoperative Therapie durchgeführt, bei der zusätzlich zu einem standardmäßig verwendeten Steroidarzneimittel (1 %iges Prednisolonacetat) Optisol® AT als Augenarzneimittel verwendet wurde. Die Verabreichung erfolgte eine Woche lang 3- bis 4-mal täglich jeweils 1 Tropen. Bereits am dritten postoperativen Tag erfolgte die Entlassung der Patientin, das Transplantat war überraschend klar und das neue Hornhautepithel bereits vollständig nachgewachsen. Dies ist insbesondere im Hinblick auf das Alter der Patientin als auch für eine Re-Keratoplastik ungewöhnlich schnell.

### Beispiel 2

Bei einem Patienten mit folgenden Refraktionsdaten wurde eine Lasik-Behandlung durchgeführt:
rechtes Auge: -3.75 0.00 25=0.8 /
linkes Auge: -2.25 -2.00 158°=0.7.

Nach der Lasik-Behandlung ergaben sich folgende Daten:
rechtes Auge: 0.00 -0.25 175°=1.6 /
linkes Auge: +0.25 -0.25 158°=1.6.

Die Nachbehandlung erfolgte wiederum mit Optisol® AT für eine Woche mit der Verabreichung von 3- bis 4-mal täglich je 1 Tropfen.

Im Vergleich zu den üblichen Behandlungen mit BSS ("Balanced Salt Solution") waren sowohl die Schmerzen postoperativ als auch der biomikroskopische Spaltlampenbefund am nächsten wie den darauffolgenden Tagen derart verbessert, daß nicht mehr erkennbar war, ob die Patientin behandelt worden war. Der biomikroskopische Befund war besser als der, der in der Regel durch Verabreichung von Steroiden und relativ hoch dosierten Antibiotika während der postoperativen Phase erreicht werden kann.

## Patentansprüche

1. Verwendung eines Gemisches, welches zumindest ein Kohlenhydrat, zumindest eine Aminosäure, zumindest ein Elektrolyt, Chondroitinsulfat und gegebenenfalls weitere übliche Hilfsstoffe enthält, zur Herstellung einer wässrigen Lösung oder Suspension, welche für das Nachwachsen von Hornhautepithel bei der Nachbehandlung von refraktiver Hornhautchirurgie oder bei der Nachbehandlung von Homhauttransplantationen 3- bis 4-mal täglich mit jeweils einem Tropfen zu verabreichen ist.

2. Verwendung nach Anspruch 1, wobei das Gemisch zusätzlich eine oder mehrere Substanzen enthält, ausgewählt aus einem oder mehreren Vitaminen, einer oder mehreren Eisenverbindungen, L-Hydroxyprolin, Adenosin, Inosin, Adenin, Lactoferrin, Lactoperoxidase, und einem Puffersystem.

## Claims

1. Use of a mixture which contains at least one carbohydrate, at least one amino acid, at least one electrolyte, chondroitin sulphate and optionally further usual additives, for the production of an aqueous solution or suspension which is to be administered 3 to 4 times daily with a respective drop for the re-growth of corneal epithelium in the aftertreatment of refractive corneal surgery or in the aftertreatment of corneal transplants.

2. Use according to claim 1 wherein the mixture additionally contains one or more substances selected from one or more vitamins, one or more iron compounds, L-hydroxyproline, adenosine, inosine, adenine, lactoferrin, lactoperoxidase, and a buffer system.

## Revendications

1. Utilisation d'un mélange qui contient au moins un hydrate de carbone, au moins un acide aminé, au moins un électrolyte, du sulfate de chondroïtine et le cas échéant d'autres adjuvants habituels, pour la fabrication d'une solution aqueuse ou d'une suspension qui doit être administrée à raison d'une goutte 3 à 4 fois par jour pour la régénération de l'épithélium cornéen pendant la postcure d'une chirurgie réfractive de l'épithélium cornéen ou pendant la postcure de transplantations de l'épithélium cornéen.

2. Utilisation selon la revendication 1, le mélange contenant en plus une ou plusieurs substances sélectionnées parmi une ou plusieurs vitamines, un ou plusieurs composés du fer, la L-hydroxyproline, l'adénosine, l'inosine, l'adénine, la lactoferrine, la lactoperoxydase et un système tampon.
